# EUROPEAN PATENT APPLICATION

(11) **EP 4 806 902 A1**
(43) Date of publication of application: **16.09.2026**
(21) Application number: 24887342.4
(22) Date of filing: 21.05.2024
(51) Int. Cl.: C07H 19/048, C07H 1/06, A61K 31/706, A61P 9/00, A23L 33/13, A61K 8/60, A61Q 19/00

(54) **AMORPHOUS FORM OF REDUCED BETA-NICOTINAMIDE MONONUCLEOTIDE CALCIUM SALT, AND PREPARATION METHOD AND USE THEREFOR**

(30) Priority: 10.11.2023 CN 202311499958
(71) Applicant: EffePharm (Shanghai) Co., Ltd., Shanghai 201601 (CN)
(72) Inventor: GAO, Xing, Shanghai 201601 (CN); YU, Jianjun, Shanghai 201601 (CN); SHEN, Qiang, Shanghai 201601 (CN); XU, Qinyuan, Shanghai 201601 (CN); DING, Yunkang, Shanghai 201601 (CN); LING, Debin, Shanghai 201601 (CN); XUAN, Lulu, Shanghai 201601 (CN); LI, Yang, Shanghai 201601 (CN); LIU, Jialing, Shanghai 201601 (CN)
(74) Representative: Lavoix
(86) International application number: PCT/CN2024/094493
(87) International publication number: WO 2025/097718

(57) **Abstract**

The invention relates to a compound NMNH calcium salt, in particular to an amorphous form of a reduced β-nicotinamide mononucleotide calcium salt, a preparation method therefor, a use thereof as a pharmaceutical ingredient, a health care product ingredient and a cosmetic ingredient or as a food additive, and a formulation containing the salt, belonging to the field of medicines, health care products, cosmetics and food additives. Specifically, described in the invention is a NMNH calcium salt amorphous compound. The compound shows long-term continuous stability, and compared with NMNH disodium salt, has significantly superior oxidation resistance and stability, and anti-hygroscopic properties, and is more beneficial to long-term storage, promotion and market application. The NMNH calcium salt amorphous compound is simple in preparation process, easy to control and suitable for large-scale production.

## Description

### Technical Field

The present invention relates to the field of chemical raw materials for pharmaceuticals, health products, cosmetics, and food additives, specifically to the amorphous form of reduced β-nicotinamide mononucleotide calcium salt, and a preparation method and a use thereof.

### Background Art

As one of the most popular molecules in the field of anti-aging, nicotinamide adenine dinucleotide (NAD⁺) has undoubtedly become the centerpiece of successive anti-aging substances. NAD⁺ is an essential coenzyme required for over 500 enzymatic reactions and is well-known for its role in oxidation and reduction (Ansari and Raghava, 2010; Rajman et al., 2018; Stein and Imai, 2012). Increasing research indicates that elevating NAD⁺ levels can significantly improve multi-organ functions, including liver function, kidney function, heart function, and skeletal muscle function (*Canto et al., 2012; Mills et al., 2016; Rajman et al., 2018*). NAD⁺ can be synthesized using tryptophan in the de novo biosynthesis pathway, nicotinic acid (NA) in the Preiss-Handler pathway, and nicotinamide (NAM), nicotinamide riboside (NR), and nicotinamide mononucleotide (NMN) in the salvage pathway (*Canto et al., 2015; Chiarugi et al., 2012; Johnson and Imai, 2018*). In particular, as key intermediates of NAD⁺, NAM, NR, and NMN have been extensively studied for their potential therapeutic effects in numerous mouse disease models (Mills et al., 2016). Among them, NMN is considered the most suitable NAD+ precursor currently, and NMN is experiencing strong global market demand, being highly favored by consumers.

NMNH (molecular structure shown as Formula (A)) has the Chinese name "reduced nicotinamide mononucleotide" or "reduced β-nicotinamide mononucleotide." It is the reduced form of NMN and serves as a novel precursor for NAD⁺ supplementation, exhibiting superior NAD⁺-promoting effects compared to NMN, along with other biological functions such as enhancing cellular antioxidant capacity, reducing fat accumulation, decreasing inflammatory responses, and inhibiting tumor cell growth. It is a health-promoting reagent with significant commercial potential (WO2021098725A1).

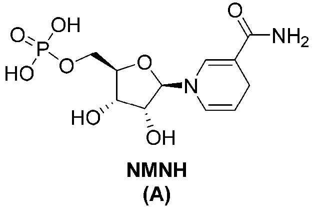

NMNH is the reduced form of NMN, sensitive to air, easily oxidized, and unstable, making it unsuitable for long-term storage and market promotion. WO2023160405(A1) reported the NMNH disodium salt compound and its crystalline and amorphous forms. When exposed in a stability test chamber at 25°C and 65% RH, the NMNH disodium salt amorphous powder turned into an oil after 1 day, with purity decreasing from 99.30% to 99.02%, while the NMNH disodium salt crystalline Form A solid showed a purity decrease from 99.33% to 99.01% after 5 days. Such stability fails to meet the shelf-life requirements for commercial products, hindering market promotion.

Therefore, there is still an urgent need in the art to develop new NMNH salt forms with improved stability, better suitability for long-term storage, and enhanced market promotion.

### Summary of the Invention

The present invention aims to provide a new NMNH salt form with improved stability and better suitability for long-term storage, specifically relating to the amorphous form of reduced β-nicotinamide mononucleotide calcium salt, its preparation method(s), and its use(s).

In a first aspect of the present invention, an amorphous compound of a reduced β-nicotinamide mononucleotide calcium salt as shown in Formula (I) is provided,

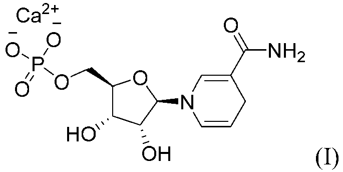

The amorphous form is selected from the group consisting of: amorphous A, amorphous B, amorphous C, or amorphous D.

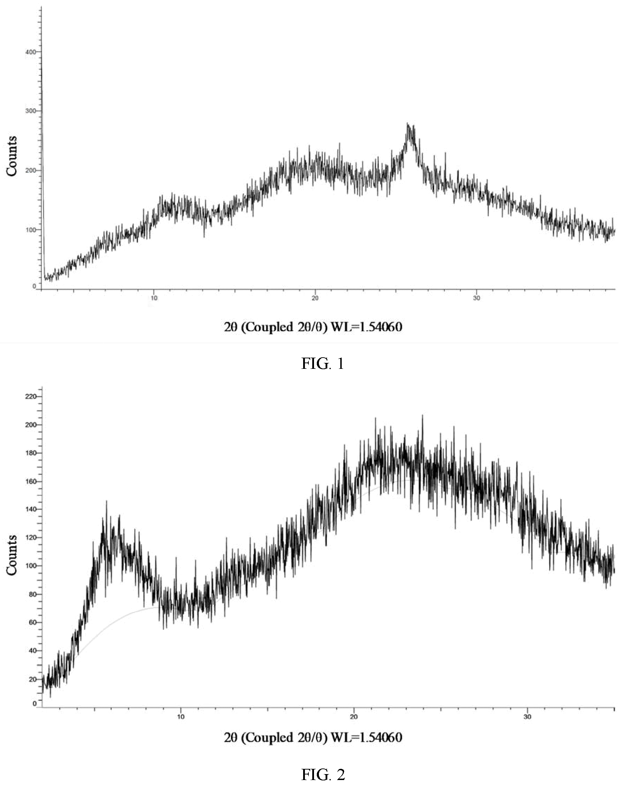

In another preferred embodiment, an XRPD pattern of the amorphous A is substantially as characterized in FIG. 1.

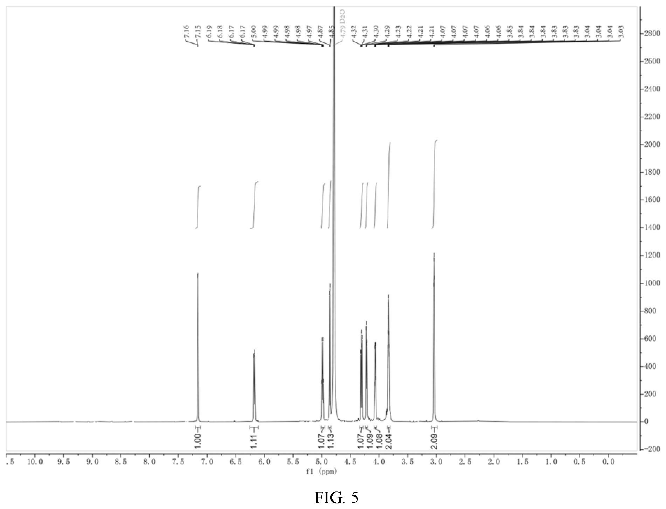

In another preferred embodiment, a ¹H NMR spectrum of the amorphous A is substantially as characterized in FIG. 5.

In another preferred embodiment, an XRPD pattern of the amorphous B is substantially as characterized in FIG. 2.

In another preferred embodiment, a ¹H NMR spectrum of the amorphous B is substantially as characterized in FIG. 5.

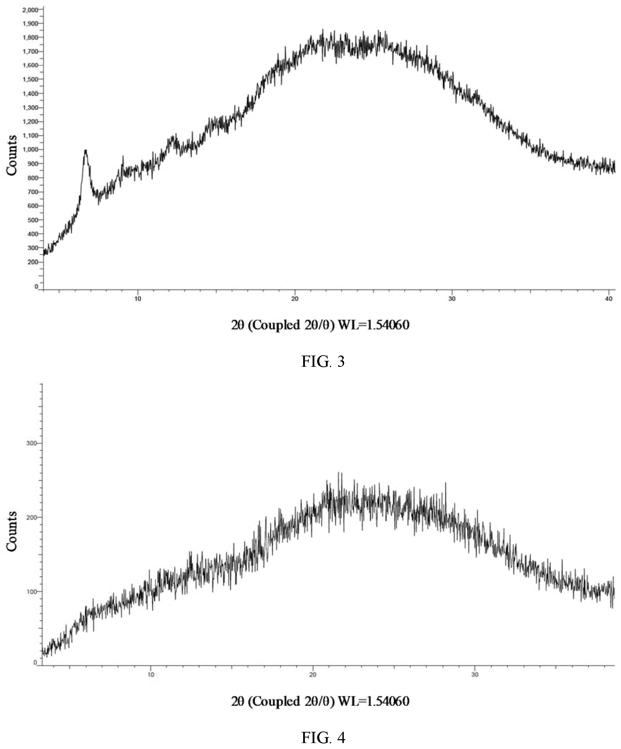

In another preferred embodiment, a XRPD pattern of the amorphous C is substantially as characterized in FIG. 3.

In another preferred embodiment, a ¹H NMR spectrum of the amorphous C is substantially as characterized in FIG. 5.

In another preferred embodiment, an XRPD pattern of the amorphous D is substantially as characterized in FIG. 4.

In another preferred embodiment, a ¹H NMR spectrum of the amorphous D is substantially as characterized in FIG. 5.

In a second aspect of the present invention, a preparation method for the amorphous compound set forth in the first aspect is provided; the method comprises the following steps:
1) adding a reduced β-nicotinamide mononucleotide calcium salt into a first solvent to obtain a solution containing the reduced β-nicotinamide mononucleotide calcium salt, or generating in situ a solution of the reduced β-nicotinamide mononucleotide calcium salt in a reaction mixture;
2) adding the solution obtained in step 1) into a second solvent to precipitate a solid to obtain the amorphous compound of the reduced β-nicotinamide mononucleotide calcium salt; or performing freeze drying or spray drying on the solution obtained in step 1) to obtain the amorphous compound of the reduced β-nicotinamide mononucleotide calcium salt; or beating the solid of the reduced β-nicotinamide mononucleotide calcium salt in the second solvent to obtain the amorphous compound of the reduced β-nicotinamide mononucleotide calcium salt according to claim 1.

In another preferred embodiment, the method performs the preparation by any one of the following methods (I) to (IV):
(I) Preparation of the amorphous A
   I-1) providing the solution of the reduced β-nicotinamide mononucleotide calcium salt formed in the first solvent;
   I-2) adding the solution obtained in step I-1) into the second solvent, drying the precipitated solid at 30-45°C to obtain the amorphous A;
   wherein the first solvent is selected from: water; the second solvent is selected from: methanol, or a mixed solvent of methanol and water;
(II) Preparation of the amorphous B
   II-1) providing the solution of the reduced β-nicotinamide mononucleotide calcium salt formed in the first solvent;
   II-2) adding the solution obtained in step II-1) into the second solvent, drying the precipitated solid at 30-45°C to obtain the amorphous B;
   wherein the first solvent is selected from: water; the second solvent is selected from: ethanol, or a mixed solvent of ethanol and water;
(III) Preparation of the amorphous C
   III-1) providing the solution of the reduced β-nicotinamide mononucleotide calcium salt formed in the first solvent;
   III-2) adding the solution obtained in step III-1) into the second solvent, drying the precipitated solid at 30-45°C to obtain the amorphous C;
   wherein the first solvent is selected from: water; the second solvent is selected from: acetone, or a mixed solvent of acetone and water;
(IV) Preparation of the amorphous D
   IV-1) providing the solution of the reduced β-nicotinamide mononucleotide calcium salt formed in the first solvent;
   IV-2) performing a drying treatment on the solution obtained in step IV-1) to obtain the amorphous D;
   wherein the drying treatment comprises: freeze drying, spray drying, vacuum drying under reduced pressure, rotary evaporation drying, etc.

In another preferred embodiment, the first solvent and the second solvent are the same or different, and are independently selected from the group consisting of: water, acetonitrile, tetrahydrofuran, methyl tert-butyl ether, 2-methyltetrahydrofuran, dichloromethane, N,N-dimethylformamide, N,N-dimethylacetamide, N,N-diethylformamide, dimethyl sulfoxide, ethyl acetate, isopropyl acetate, ketone solvents, alcohol solvents, or combinations thereof.

In another preferred embodiment, the ketone solvent is selected from the group consisting of: acetone, 2-butanone, methyl isobutyl ketone, methyl tert-butyl ketone, 3-methyl-2-butanone, or combinations thereof.

In another preferred embodiment, the alcohol solvent is selected from the group consisting of: methanol, ethanol, n-propanol, isopropanol, n-butanol, isobutanol, n-pentanol, or combinations thereof.

In another preferred embodiment, the solution of the reduced β-nicotinamide mononucleotide calcium salt formed in the first solvent is provided by the following method: adding the reduced β-nicotinamide mononucleotide calcium salt to the first solvent to obtain a solution containing the reduced β-nicotinamide mononucleotide calcium salt, or generating a reduced β-nicotinamide mononucleotide calcium salt solution in situ in a reaction mixture.

In a third aspect of the present invention, a composition is provided, the composition comprises: (a) the amorphous compound according to the first aspect, and (b) a pharmaceutically acceptable excipient or carrier, or a health product acceptable excipient or carrier, or a cosmetically acceptable excipient or carrier, or a food acceptable excipient or carrier.

In another preferred embodiment, the composition is selected from the group consisting of: a pharmaceutical composition, a health product composition, a cosmetic composition, or a food composition.

In another preferred embodiment, the pharmaceutical composition comprises: (a) the amorphous compound as set forth in the first aspect, and (b) a pharmaceutically acceptable excipient or carrier.

In another preferred embodiment, the dosage form of the pharmaceutical composition is selected from the group consisting of: oral formulation, injectable formulation, respiratory administration formulation, dermal administration formulation, mucosal administration formulation, cavity administration formulation, and the like.

In another preferred embodiment, the health product composition comprises: (a) the amorphous compound as set forth in the first aspect, and (b) an excipient or carrier acceptable for health products.

In another preferred embodiment, the cosmetic composition comprises: (a) the amorphous compound as set forth in the first aspect, and (b) an cosmetically acceptable excipient or carrier.

In another preferred embodiment, the cosmetic composition comprises cosmetics used for purposes selected from the group consisting of: skin cosmetics, hair cosmetics, beauty cosmetics, and special function cosmetics.

In another preferred embodiment, the food composition comprises: (a) the amorphous compound as set forth in the first aspect, and (b) an excipient or carrier acceptable for food.

In a fourth aspect of the present invention, a use of the amorphous compound as set forth in the first aspect for the preparation of a medicament, a health product, a cosmetic or a food additive is provided.

In another preferred embodiment, the drug is used to protect the optic nerve, improve retinal damage, prevent/treat hair loss, prevent/improve cardiovascular and cerebrovascular diseases, inhibit renal tubular damage and aging, prevent liver fibrosis, improve fatty liver disease, alleviate dry eye symptoms, repair kidney damage, prevent diabetes/nephropathy, improve sarcopenia symptoms in the elderly, treat chronic inflammation, alleviate the condition of patients with polycystic ovary syndrome, prevent/delay glaucoma, reduce neuroinflammation, mitigate the cardiac toxicity of anthracycline chemotherapeutic drugs, aid in stroke recovery, and prevent/treat heart failure in the elderly.

In another preferred embodiment, the health supplement is used to slow cellular aging, delay female reproductive aging, enhance fertility, improve menopausal symptoms, enhance male sexual function, improve sleep, soothe emotions, boost energy, improve cardiovascular function, enhance cardiovascular health, boost immunity, improve sub-health conditions, prevent tumors, and prevent Alzheimer's disease.

In another preferred embodiment, the cosmetic is used to improve damaged cell function, enhance skin/hair quality, prevent/treat skin photoaging, maintain skin softness and elasticity, and delay skin aging.

In another preferred embodiment, the food additive is used to improve appetite, enhance digestive function, promote metabolism, promote hair/nail growth, and enhance nutritional value.

It should be understood that within the scope of the present invention, the above technical features of the present invention and the technical features specifically described below (e.g., in the embodiments) can be combined with each other to form new or preferred technical solutions. Due to space limitations, they are not exhaustively listed herein.

### Brief Description of the Drawings

FIG. 1 shows an XRPD pattern of NMNH calcium salt amorphous form A.
FIG. 2 shows an XRPD pattern of NMNH calcium salt amorphous form B.
FIG. 3 shows an XRPD pattern of NMNH calcium salt amorphous form C.
FIG. 4 shows an XRPD pattern of NMNH calcium salt amorphous form D.
FIG. 5 shows a ¹H NMR spectrum of the NMNH calcium salt amorphous form.

### Description of the Embodiments

Through extensive and in-depth research, the inventors unexpectedly developed a specific salt of NMNH for the first time, and the salt can be NMNH calcium salt. The studies of the present invention demonstrate that the amorphous form of NMNH calcium salt exhibits excellent stability. Compared to the crystalline and amorphous forms of NMNH disodium salt, the amorphous form of NMNH calcium salt shows long-term sustained stability and resistance to moisture absorption, making it more suitable for long-term storage and market promotion. Furthermore, the amorphous compound of the present invention meets the shelf-life requirements for commercial products and is suitable for use in pharmaceutical compositions, health supplements, cosmetics, food additives, and the like. On this basis, the inventors completed the present invention.

### Terminology

Unless otherwise defined, all technical and scientific terms used herein have the same meaning as commonly understood by a person of ordinary skill in the art to which the present invention belongs.

### NMNH calcium salt

As used herein, the terms "reduced β-nicotinamide mononucleotide calcium salt," "β-dihydronicotinamide mononucleotide calcium salt," "dihydronicotinamide mononucleotide calcium salt," "reduced nicotinamide mononucleotide calcium salt," "reduced NMN calcium salt," "NMNH calcium salt," and "NMNH-Ca" are used interchangeably and all refer to the salt formed by reduced β-nicotinamide mononucleotide and calcium ions, with the structure shown in Formula (I). It should be understood that this term includes amorphous forms, hydrates, solvates, solvate-hydrates, and anhydrates.

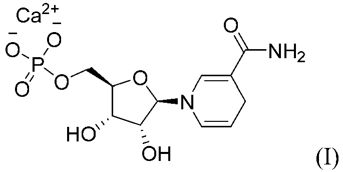

### Use of the Reduced β-Nicotinamide Mononucleotide Calcium Salt of the Present Invention

The present invention provides the use of the amorphous NMNH calcium salt compound, which is highly efficient and broad-spectrum, and can be used in pharmaceutical compositions, health products, cosmetics, food additives, and the like.

### Compared with the existing technology, the beneficial effects of the present invention are as follows:

(1) The amorphous solid of the compound of Formula (I) of the present invention, compared with the crystalline and amorphous solids of NMNH disodium salt, exhibits better antioxidant properties and stability, lower hygroscopicity, and is more favorable for subsequent formulation processes, long-term storage, and market promotion.
(2) The preparation method of the amorphous solid of the compound of Formula (I) of the present invention is simple and suitable for industrial production.
(3) The amorphous solid of the compound of Formula (I) of the present invention can be used in pharmaceutical compositions, health products, cosmetics, food additives, and the like.

The present invention is further described below in conjunction with specific examples. It should be understood that these examples are only used to illustrate the present invention, and are not intended to limit the scope of the present invention. The experimental methods in the following examples, for which specific conditions are not specified, are generally conducted under conventional conditions or according to the conditions recommended by the manufacturer. Unless otherwise stated, percentages and parts are by weight.

Unless otherwise defined, all professional and scientific terms used herein have the same meaning as those familiar to a person skilled in the art. Furthermore, any methods and materials similar or equivalent to those described can be applied to the methods of the present invention.

The experimental materials and reagents used in the following examples, unless otherwise specified, can be obtained from commercially available sources.

### Test Methods:

XRPD (X-ray Powder Diffraction) Pattern Determination Method: Bruker D2 Phaser X-ray Powder Diffractometer; Radiation Source Cu (1.54060 Å).

Measurement variations associated with the results of this type of X-ray powder diffraction Asc diffraction analysis arise from several factors, including: (a) errors in sample preparation (e.g., sample height), (b) instrument errors, (c) calibration differences, (d) operator errors (including those occurring during peak position determination), and (e) material properties (e.g., preferred orientation errors). Calibration errors and sample height errors often lead to displacement of all peaks in the same direction. When using a flat holder, small differences in sample height can lead to significant displacement in XRPD peak positions. Systematic studies show that a 1 mm difference in sample height can result in a peak shift of up to 1° in 2θ. These displacements can be identified from the XRPD pattern and can be corrected by compensating for the displacement (applying a systematic calibration factor to all peak position values) or by recalibrating the instrument. As mentioned above, by applying a systematic calibration factor to align peak positions, measurement errors from different instruments can be corrected.

### Example 1. Preparation of NMNH Calcium Salt Aqueous Solution

To 10 liters of saturated sodium bicarbonate aqueous solution, 1.7 kg of β-NMN and 0.94 kg of sodium dithionite were added, stirred at room temperature overnight, and filtered to obtain a clear solution. The pH of the clear solution was adjusted to 3-4 with 37% hydrochloric acid. The solution was desalted using electrodialysis until the conductivity of the solution decreased to 50-100 µS. The pH of the solution was adjusted to 10 with calcium hydroxide; alternatively, 0.57 kg of calcium chloride was added, and the pH was adjusted to 10 with sodium hydroxide. An NMNH calcium salt aqueous solution was obtained, containing approximately 1.71 kg of NMNH calcium salt (solution purity determined by HPLC to be 94.5%).

### Example 2. Preparation of NMNH Calcium Salt Amorphous A Solid

500 ml of NMNH calcium salt aqueous solution was added dropwise to 2 L of stirring methanol. After the addition was complete, the mixture was stirred for 1 hour, filtered, and dried at 30-45°C to obtain 80.3 g of NMNH calcium salt amorphous A product, with a yield of 85.0% and a purity of 99.5%.

The obtained solid was subjected to X-ray powder diffraction testing. The XRPD pattern of the NMNH calcium salt amorphous A is substantially as shown in FIG. 1, and the ¹H NMR spectrum is substantially as shown in FIG. 5.

### Example 3. Preparation of NMNH Calcium Salt Amorphous B Solid

500 ml of NMNH calcium salt aqueous solution was added dropwise to 2 L of stirring ethanol. After the addition was complete, the mixture was stirred for 1 hour, filtered, and dried at 30-45°C to obtain 82.6 g of NMNH calcium salt amorphous B product, with a yield of 87.5% and a purity of 99.2%.

The obtained solid was subjected to X-ray powder diffraction testing. The XRPD pattern of the NMNH calcium salt amorphous B is substantially as shown in FIG. 2, and the ¹H NMR spectrum is substantially as shown in FIG. 5.

### Example 4. Preparation of NMNH Calcium Salt Amorphous C Solid

500 ml of NMNH calcium salt aqueous solution was added dropwise to 2 L of stirring acetone. After the addition was complete, the mixture was stirred for 1 hour, filtered, and dried at 30-45°C to obtain 83.5 g of NMNH calcium salt amorphous C product, with a yield of 88.4% and a purity of 99.1%.

The obtained solid was subjected to X-ray powder diffraction testing. The XRPD pattern of the NMNH calcium salt amorphous C is substantially as shown in FIG. 3, and the ¹H NMR spectrum is substantially as shown in FIG. 5.

### Example 5. Preparation of NMNH Calcium Salt Amorphous D Solid

10.0 g of NMNH calcium salt amorphous A solid was dissolved in 100 ml of purified water and subjected to freeze-drying to obtain 9.8 g of NMNH calcium salt amorphous D product, with a yield of 98% and a purity of 99.5%.

The obtained solid was subjected to X-ray powder diffraction testing. The XRPD pattern of the NMNH calcium salt amorphous D is substantially as shown in FIG. 4, and the ¹H NMR spectrum is substantially as shown in FIG. 5.

### Example 6. Preparation of NMNH Calcium Salt Amorphous D Solid

10.0 g of NMNH calcium salt amorphous A solid was dissolved in 100 ml of purified water and subjected to spray-drying to obtain 9.0 g of NMNH calcium salt amorphous D product, with a yield of 90% and a purity of 99.5%.

The obtained solid was subjected to X-ray powder diffraction testing. The XRPD pattern of the NMNH calcium salt amorphous D is substantially as shown in FIG. 4, and the ¹H NMR spectrum is substantially as shown in FIG. 5.

### Example 7. Stability Comparison of NMNH Calcium Salt Amorphous Solids A/B/C/D with NMNH Disodium Salt Crystalline Form A and Amorphous Solid

The NMNH calcium salt amorphous solids A/B/C/D were each placed openly in a stability test chamber at 25°C and 65% RH to evaluate their stability, yielding the data shown in Table 1 and Table 2.

**Table 1: Stability Comparison of NMNH Calcium Salt Amorphous Solids with NMNH Disodium Salt Crystalline Form A and Amorphous Solid (25°C, 65% RH)**

| Time | Purity (HPLC, area%) | | | | | |
|---|---|---|---|---|---|---|
| | NMNH disodium salt | | NMNH calcium salt | | | |
| | Crystal form A (powder) | Amorphous form (powder) | Amorphous form A (powder) | Amorphous form B (powder) | Amorphous form C (powder) | Amorphous form D (powder) |
| 0 day | 99.33 | 99.30 | 99.51 | 99.28 | 99.14 | 99.50 |
| 1 day | 99.27 | 99.02 (oil) | / | / | / | / |
| 5 days | 99.01 | / | 99.50 | 99.28 | 99.14 | 99.49 |
| 60 days | / | / | 99.50 | 99.28 | 99.13 | 99.49 |

**Table 2: Hygroscopicity Study of NMNH Calcium Salt Amorphous Solids (25°C, 65% RH)**

| Time | Water content of NMNH calcium salt amorphous solid (KF) | | | |
|---|---|---|---|---|
| | Amorphous form A | Amorphous form B | Amorphous form C | Amorphous form D |
| 0 day | 0.8% | 1.3% | 1.5% | 1.9% |
| 5 days | 0.8% | 1.3% | 1.6% | 1.9% |
| 60 days | 0.9% | 1.3% | 1.5% | 1.9% |

According to WO2023160405 (A1), when NMNH disodium salt is stored in the air, both crystal forms B and C absorb water and transform into crystal form A. After saturation with water, the water content of crystal form A is 19%-30% (high water content), and the purity drops from 99.33% to 99.01% after 5 days. The amorphous solid of NMNH disodium salt is more unstable in the air. After 1 day of storage, it absorbs water from powder to oil, and the purity drops from 99.30% to 99.02%.

It can be seen from Table 1 and Table 2 that after 60 days of storage, the amorphous solid of NMNH calcium salt is still a solid powder, and the purity and water content are almost unchanged. That is, the amorphous solid of NMNH calcium salt can be placed in the open air at 25°C and 65% RH for no less than 60 days, and the purity and water content can be continuously stable. However, both the crystalline form A and the amorphous form of NMNH disodium salt are unstable and easily absorb water, which is extremely unfavorable for storage, cannot meet the shelf life of the product, and is difficult to market and promote. It can be seen that the stability and hygroscopicity resistance of the amorphous solid of NMNH calcium salt are significantly improved, which is conducive to the subsequent preparation process operation and long-term continuous and stable storage, and is convenient for market promotion.

All documents mentioned in the present invention are cited as references in the present application, just as if each document was cited as reference individually. In addition, it should be understood that after reading the above teachings of the present invention, a person skilled in the art can make various changes or modifications to the present invention, and these equivalent forms also fall within the scope defined by the appended claims of the present application.

## Claims

1. An amorphous compound of a reduced β-nicotinamide mononucleotide calcium salt as shown in formula (I), 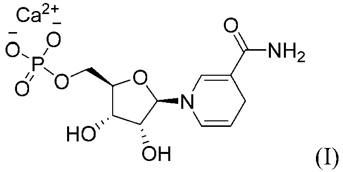 **characterized in that** the amorphous is selected from the group consisting of: amorphous A, amorphous B, amorphous C, or amorphous D.

2. The amorphous according to claim 1, **characterized in that** an XRPD spectrum of the amorphous A is substantially characterized as shown in FIG. 1.

3. The amorphous according to claim 1, **characterized in that** an XRPD spectrum of the amorphous B is substantially characterized as shown in FIG. 2.

4. The amorphous according to claim 1, **characterized in that** an XRPD spectrum of the amorphous C is substantially characterized as shown in FIG. 3.

5. The amorphous according to claim 1, **characterized in that** an XRPD spectrum of the amorphous D is substantially characterized as shown in FIG. 4.

6. A preparation method of the amorphous compound according to claim 1, **characterized in that** the method comprises the following steps:
1) adding a reduced β-nicotinamide mononucleotide calcium salt into a first solvent to obtain a solution containing the reduced β-nicotinamide mononucleotide calcium salt, or generating in situ a solution of the reduced β-nicotinamide mononucleotide calcium salt in a reaction mixture;
2) adding the solution obtained in step 1) into a second solvent to precipitate a solid to obtain the amorphous compound of the reduced β-nicotinamide mononucleotide calcium salt; or performing freeze drying or spray drying on the solution obtained in step 1) to obtain the amorphous compound of the reduced β-nicotinamide mononucleotide calcium salt; or beating the solid of the reduced β-nicotinamide mononucleotide calcium salt in the second solvent to obtain the amorphous compound of the reduced β-nicotinamide mononucleotide calcium salt according to claim 1.

7. The method according to claim 6, **characterized in that** the method performs the preparation by any one of the following methods (I) to (IV):
(I) Preparation of the amorphous A
I-1) providing the solution of the reduced β-nicotinamide mononucleotide calcium salt formed in the first solvent;
I-2) adding the solution obtained in step I-1) into the second solvent, drying the precipitated solid at 30-45°C to obtain the amorphous A;
wherein the first solvent is selected from: water; the second solvent is selected from: methanol, or a mixed solvent of methanol and water;
(II) Preparation of the amorphous B
II-1) providing the solution of the reduced β-nicotinamide mononucleotide calcium salt formed in the first solvent;
II-2) adding the solution obtained in step II-1) into the second solvent, drying the precipitated solid at 30-45°C to obtain the amorphous B;
wherein the first solvent is selected from: water; the second solvent is selected from: ethanol, or a mixed solvent of ethanol and water;
(III) Preparation of the amorphous C
III-1) providing the solution of the reduced β-nicotinamide mononucleotide calcium salt formed in the first solvent;
III-2) adding the solution obtained in step III-1) into the second solvent, drying the precipitated solid at 30-45°C to obtain the amorphous C;
wherein the first solvent is selected from: water; the second solvent is selected from: acetone, or a mixed solvent of acetone and water;
(IV) Preparation of the amorphous D
IV-1) providing the solution of the reduced β-nicotinamide mononucleotide calcium salt formed in the first solvent;
IV-2) performing a drying treatment on the solution obtained in step IV-1) to obtain the amorphous D;
wherein the drying treatment comprises: freeze drying, spray drying, vacuum drying under reduced pressure, rotary evaporation drying, etc.

8. The method according to claim 6, **characterized in that** the first solvent and the second solvent are the same or different, and are independently selected from the group consisting of: water, acetonitrile, tetrahydrofuran, methyl tert-butyl ether, 2-methyltetrahydrofuran, dichloromethane, N,N-dimethylformamide, N,N-dimethylacetamide, N,N-diethylformamide, dimethyl sulfoxide, ethyl acetate, isopropyl acetate, ketone solvents, alcohol solvents, or combinations thereof.

9. A composition, **characterized in that** the composition comprises: (a) the amorphous compound according to claim 1, and (b) a pharmaceutically acceptable excipient or carrier, or a health product acceptable excipient or carrier, or a cosmetically acceptable excipient or carrier, or a food acceptable excipient or carrier.

10. A use of the amorphous compound according to claim 1, **characterized in that** the amorphous compound is used for the preparation of a medicament, or a health product, or a cosmetic, or a food additive.
